# EUROPEAN PATENT APPLICATION

(11) **EP 1 743 594 A1**
(43) Date of publication of application: **17.01.2007**
(21) Application number: 05106481.4
(22) Date of filing: 14.07.2005
(51) Int. Cl.: A61C 1/08, A61C 19/00, A61G 15/10, A61G 15/16, A61B 19/00

(54) **Computer-Controlled Dental Treatment System And Method**

(71) Applicant: Cohen, Yechiel, Carmiel 21861 (IL)
(72) Inventor: Cohen, Yechiel, Carmiel 21861 (IL)
(74) Representative: Schmitz, Jean-Marie

(57) **Abstract**

A computer-controlled dental treatment system, includes: a dental chair for receiving a patient to be treated; a robotic arm carrying a dental tool holder for holding a dental tool to be used in rendering a selected dental treatment, and for manipulating the dental tool with respect to the patient's head during the dental treatment; and a control system programmable to control the robotic arm to manipulate the dental tool in accordance with the selected dental treatment to be rendered to the patient.

## Description

### FIELD AND BACKGROUND OF THE INVENTION

The present invention relates to a dental treatment system and method, and particularly to a computer-controlled dental treatment system and method.

A number of computer-based systems and methods have been proposed for aiding a dentist in performing a particular dental treatment. For example, US Patent 6,640,128 relates to a method and device for assisting the dentist in navigating a dental tool during a dental treatment; US Patent 6,602,070 discloses a technique for generating and displaying a plurality of treatment paths for selection by the dentist; US Patent 5,453,009 discloses a technique for examining and storing patient mouth data regarding possible dental diseases or problems and for generating a sequence of treatments to guide the dentist in treating the patient; and US Patents 5,562,448 and 4,997,369 relate to methods and systems for scanning the outer surface of a tooth or jaw a structure to assist the dentist in the dental treatment. All such systems, however, still require the actual treatments to be rendered by the dentist, and therefore, they not only requires the dentist to also perform routing and menial tasks, but also make the quality of the service rendered completely dependent on the skill and experience of the dentist.

### OBJECTS AND BRIEF SUMMARY OF THE PRESENT INVENTION

An object of the present invention is to provide a dental treatment system having advantages in one or more of the above respects.

According to one aspect of the present invention, there is provided a dental treatment system, comprising: a dental chair for receiving a patient to be treated, and including a head rest for receiving the head of the patient; a robotic arm carrying a dental tool holder for holding a dental tool to be used in rendering a selected dental treatment, and for manipulating the dental tool with respect to the patient's mouth during the dental treatment; and a control system programmable to control the robotic arm to manipulate the dental tool in accordance with the selected dental treatment to be rendered to the patient.

According to further features in the preferred embodiments of the invention described below, the dental chair further includes a jaw-engageable device engageable with the lower jaw of a patient on the dental chair to retain the lower jaw in a desired open position during a dental treatment.

According to another preferred feature in the described embodiment, the robotic arm further includes a hand grip graspable by a dentist for manually manipulating the dental tool holder, and the dental tool held thereby, independently of the control system.

According to still further preferred features, the system further includes a dental tray for holding various dental tools for convenient access by the dental tool holder carried by the robotic arm.

In the described preferred embodiment, the control system is also programmable to control the robotic arm to automatically engage its dental tool holder with the appropriate dental tool carried by the tray in accordance with the selected dental treatment to be rendered to the patient.

According to another aspect of the present invention, there is provided a method of rendering a selected dental treatment to a patient, comprising: providing a dental chair for the patient to receive the dental treatment, and a robotic arm carrying a dental tool to be used in rendering a selected dental treatment to the patient. The method further comprises a data preparation phase, in which this is stored in a first database, patient data regarding the physical dimensions of the patient to be treated relevant to the selected dental treatment to be rendered, and there is stored in a second database, control data regarding controls to be applied to the robotic arm for rendering the selected dental treatment to the patient. The method further comprises a dental treatment phase, in which the data from the first and second databases is utilized for automatically controlling the robotic arm to manipulate the dental tool in accordance with the patient data in the first database and the control data in the second database for rendering the selected dental treatment to the respective patient on the dental chair.

Further features and advantages of the invention will be apparent from the description below.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. In these drawings:
FIG. 1 illustrates a computer-controlled dental treatment system constructed in accordance with the present invention;
FIG. 2 illustrates the workstation in the computer-controlled system of Fig. 1;
FIG. 3 is block diagram illustrating a system for gathering preliminary patient data in the data preparation phase of a dental treatment in accordance with the present invention;
FIG. 4 is a block diagram illustrating a dental treatment phase in accordance with the present invention;
FIG. 5 illustrates one technique for using or applying a reference mark in the patient's jaws during the data preparation phase, for use in controlling the manipulations of the robotic arm in the dental treatment phase; and
FIG. 6 a block diagram illustrating one method of using the system for various dental treatments.

### DESCRIPTION OF A PREFERRED EMBODIMENT

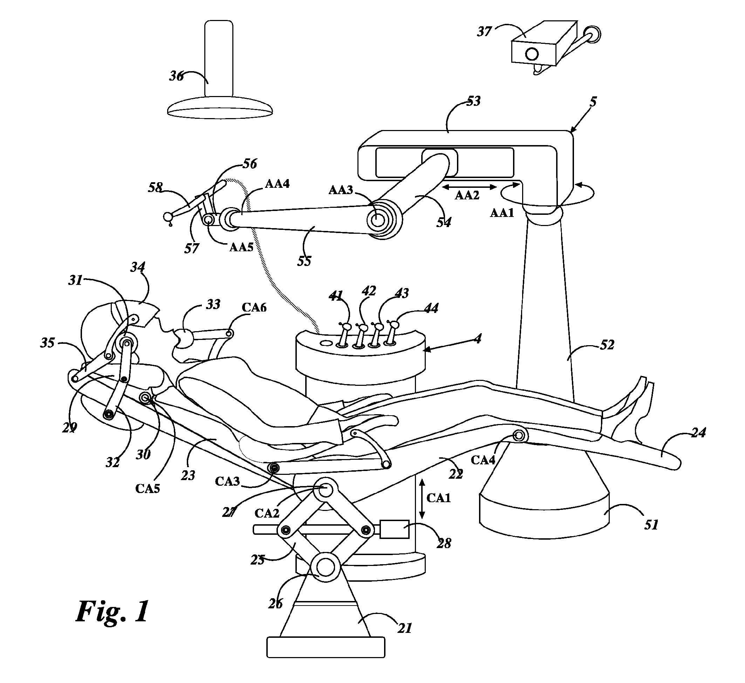

With reference to Fig. 1, there is illustrated a dental chair, generally designated 2, which may be fully or partially controlled automatically according to the specific characteristics of the patient and the specific dental treatment to be rendered. Also illustrated in Fig. 1 are a dental tray 4 for holding the various dental tools, and a robotic arm 5, which is also automatically controlled in accordance with the specific treatment to be rendered to the patient and the specific characteristics of the patient.

Thus, the dental chair 2 illustrated in Fig. 1 includes a base 21 mounting a seat 22, back rest 23 and leg rest 24 for supporting the patient in a number of different positions as may be required according to the specific characteristics of the patient and the treatment to be rendered. The seat 22, back rest 23 and leg rest 24 are mounted for vertical adjustment by a lazy tongs mechanism 25 which is pivotably mounted to the base 21 about a horizontal axis 26. The seat 22 is pivotally mounted to mechanism 25 about a pivotal axis 27. The vertical adjustment of the chair is effected by a piston-and-cylinder device 28 for driving lazy tongs mechanism 25 in one direction to lower seat 22, back rest 23 and leg rest 24, or in the opposite direction to raise the latter elements of the dental chair.

The illustrated dental chair further includes a head rest 29 rotatable about a horizontal axis 30 to engage and support the neck and head of the patient in a comfortable position. The illustrated dental chair further includes a pair of ear pads 31 carried at the ends of a pair of arms 32 selectively adjustable so as to enclose the two ears of the patient. The ear pads 31 may be used for muting noise likely to cause tension or distress during the treatment, and/or for transmitting music for relaxing the patient during the treatment. Arms 32 of the ear pads may be fixed to the dental chair so as to be effective to restrain the patient from moving his or her head during a dental treatment.

The dental chair illustrated in Fig. 1 further includes a jaw gripper device 33 which engages the lower jaw of the patient to retain it in a desired open position during a dental treatment.

The illustrated dental chair may be further equipped with a television screen 34 pivotally mounted by a pair of arms 35 so as to be pivoted to an operative position in alignment with the patient's eyes. Television screen 34 may be configured for application to the forehead of a patient to ----- the eyes of the patient on the dental chair. The television screen may be used for displaying to the patient an entertainment program to aid in relaxing the patient. It may also be used for displaying to the patient the actual operations performed by the dentist as imaged by a video camera 35 oriented to image the working area of the patient's face as illuminated by a light source 36.

The dental tray 4 is located so as to be conveniently accessible to the dentist when treating the patient. Dental tray 4 includes a plurality of holders 41 - 44 for holding various dental tools or implements that may be required during any particular dental treatment.

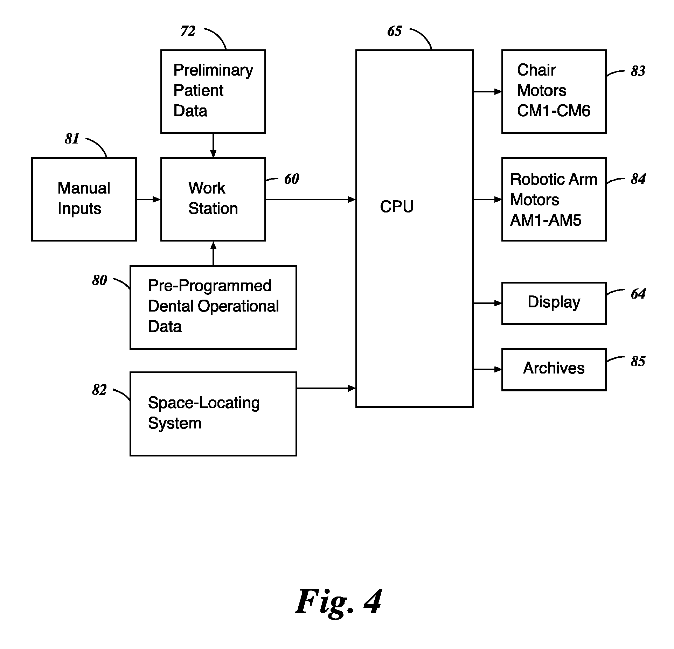

The foregoing movements of the dental chair 2 are effected by a number of electrical motors as schematically shown at CM₁-CM₆ in Fig. 4. Thus, motor CM₁ controls the piston-cylinder drive 28 for the lazy-tong mechanism 25 to move seat 22 vertically along the vertical axis CA₁; motor CM₂ pivots the seat 22 about horizontal pivot axis 27 (CA₂); motor CM₃ pivots the back rest 23 about axis CA₃; and motor CM₄ pivots the leg rest 24 about axis CA₄. An additional motor CM₅ may be provided to pivot the head rest 29 about axis 30 (CA₅); and motor CM₆ may be provided to pivot the lower-jaw grip 33 about axis CA₆.

The robotic arm 5 includes a base 51, a vertical post 52 mounted on the base, and a first horizontal arm 53 rotatably mounted to the vertical post above a vertical axis AA₁. Robotic arm 5 further includes a second horizontal arm 54 slidably mounted to horizontal arm 53 above a horizontal axis AA₂; a first pivotal arm 55 pivotally mounted to one end of horizontal arm 54 about a horizontal axis AA₃; and a second pivotal arm 56 pivotally mounted at one end to the opposite end of pivotal arm 55 about axis AA₄. A hand grip 57 is pivotally mounted to arm 56 about axis AA₅; and a tool holder 58 is clamped to hand grip 57.

It will be seen that tool holder 58 is carried by robotic arm 5 so as to permit movement of the tool holder along five (or more) axes of movements, AA₁-AA₅. Each of the foregoing axes of movement of the tool holder 58 on robotic arm 5 is provided with an electrical motor AM₁-AM₅ which is controlled, as will be described more particularly below with respect to Fig. 4, for precisely positioning and moving the tool carried by the tool holder during a dental treatment.

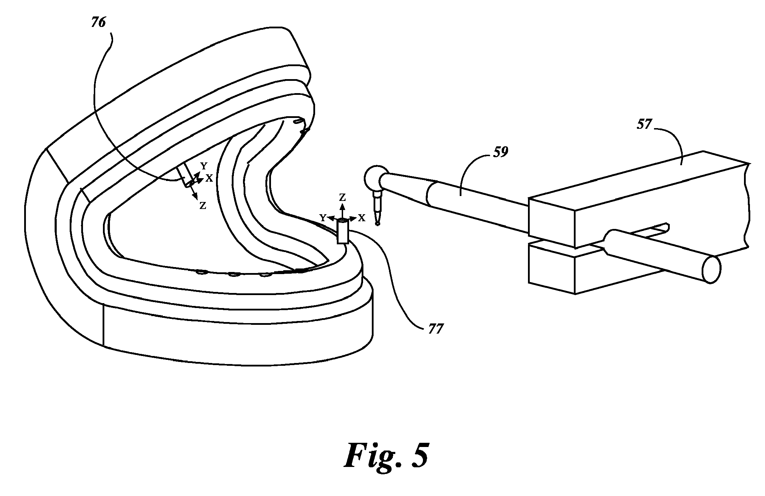

As will be also described more particularly below, hand grip 57 is adapted to carry, not only a tool holder 58 for use in the dental treatment, but also a manual probe 59, as shown in Fig. 5 for locating pre-selected reference points in the patient's mouth to serve as reference points in the control of the robotic arm 5 by the control system during the dental treatment.

The dental tray 4 holds the various dental implements that may be needed for a selected treatment. There implements are picked-up by the robotic arm 5 by means of the hand grip 57 according to the commands received from the computer. Hand grip 57, however, enables the dentist to override any computer command, whenever necessary, and to manually maneuver the implement as desired by the dentist during a particular dental operation.

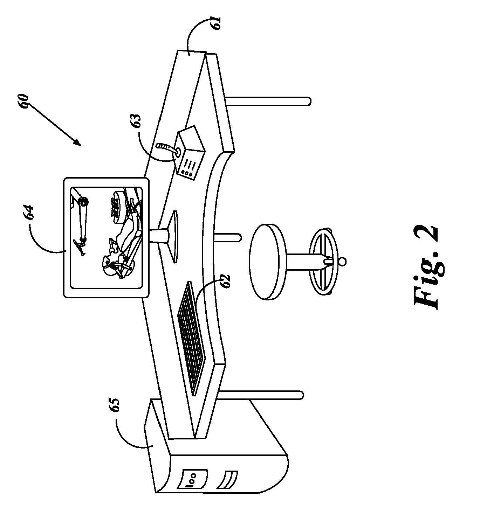

The foregoing movements of the robotic arm 5, as well as of the dental chair 2, are controlled by a computer at a workstation illustrated in Fig. 2, and therein generally designated 60. Workstation 60 includes a main frame 61 mounting various input and output devices, including a keyboard 62, a joy stick 63, and a monitor 64. The operations performed by the work station 60 are controlled by a CPU processor 65, such as a personal computer programmed to perform the various operations as will be described more particularly below.

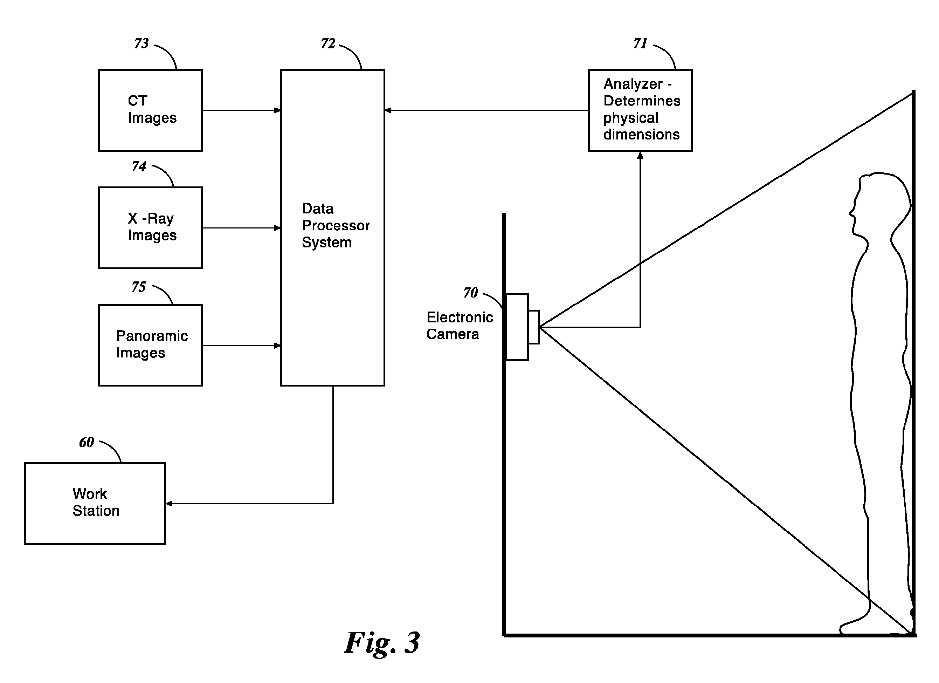

Fig. 3 diagrammatically illustrates the manner of gathering the preliminary personal data concerning the patient, including the patient's physical dimensions, as well as other data relating to the patient derived from CT, X-ray and/or panoramic images generally prepared before the specific dental treatment is to be rendered.

Thus, as shown in Fig. 3, the patient is imaged by an electronic camera 70, the output of which is analyzed by an analyzer unit 71 to produce measurements of the basic physical dimensions of the respective patient required for the dental treatment to be rendered. These measurements are inputted into a data processor 72, which also includes a CT image input 73, an X-ray image input 74, and a panoramic image input 75. Data processor 72 processes the data from inputs 71, 73, 74 and 75, and outputs the processed data to the workstation 60.

The system illustrated in Fig. 3 is thus used to gather all the relevant information concerning the particular patient to receive the dental treatment, such as personal data, CT image data, X-ray image data and panoramic image data. This data also includes images from the electronic camera 70 of the overall physical structure of the patient, and particularly of the region of the patient's head to receive the dental treatment.
Thus, as shown in Fig. 5, the patient data particularly includes the details of the physical construction of the patient's two jaws, at least one of which is to receive the dental treatment. Such structural details also include a selected reference point in each of the two jaws to serve as reference points in the control of the robotic arm 5 by the control system during an actual dental treatment. Fig. 5 illustrates a reference point 76 in the upper jaw, and a second reference point 77 in the lower jaw.

These reference points may be existing elements already present in the patient's mouth, e.g. a metal filling, a metal implant, or other machine-sensible element that can be sensed during the actual dental treatment for use as a reference with respect to tool operations to be formed during the dental treatment. If an appropriate reference point is not already present in one of the jaws, particularly the jaw to be involved in the treatment, a machine-sensible element, such a metal or magnetic pin or other market element, could be applied to the patient's mouth, preferably at a location outside that of the actual working area but sufficiently close to it to serve as an accurate reference for guiding the dental tool used in the dental operations to be performed in the respective working area.

As will be described more particularly below, workstation 60 utilizes this data, representing all the relevant information concerning the particular patient, for controlling both the dental chair 2 and the robotic arm 5 to aid the dentist in performing the particular dental operations to be rendered to the patient.

Fig. 4 diagrammatically illustrates the work station 60 of Fig. 2, the CPU processor 65 at the workstation, and the various operations performed at the workstation.

Thus, as shown in Fig. 4, the workstation 60 includes one input from the data processor 72 of Fig. 3 representing the preliminary data personal to the particular patient to receive the dental treatment. Workstation 60 includes a second input 80 representing pre-programmed data relevant to the selected dental operation. For example, the dental operation may be an implant treatment, a tool extraction, a gum treatment or various types of surgical treatments. Each such treatment is represented by a software data file setting forth the controls of the chair motors CM₁ - CM₆ and robotic arm motors AM₁ - AM₅ involved in the respective dental treatment.

The treatment program for the respective treatment to be rendered can be pre-prepared by specialists in the respective field, or can be pre-prepared by the dentists to render the treatment according to the specific condition of the patient. The treatment program may be displayed to the dentist on monitor 64 before the treatment is to be executed, as well as in real time during the operations involved in the specific treatment so that they can be clearly monitored by the dentist. The monitor thus enables the dentist to view the operations as they are performed, and thereby to closely control the treatment process.

Workstation 60 further enables manual inputs 81 by the dentist while in the workstation. Such manual inputs may include preliminary data inputted via the keyboard 62, and control data inputted by the dentist in a real-time manner during a specific dental treatment by joy stick 63. The preliminary inputted data would relate primarily to the various controls of the dental chair 2 to properly prepare the patient seated thereon for receiving the dental treatment; whereas the control data would relate primarily to the various controls of the robotic arm 5 and the manual joystick 63 in the workstation 60 according to the specific dental treatment to be rendered to the patient.

The location of the reference points 76, 77 (Fig. 5) would be included with the preliminary data personal to the particular patient as gathered by the system illustrated in Fig. 3. Alternatively, it could be manually inputted via the keyboard 62 or joystick 63.

At the beginning of a dental operation, after the patient has been properly positioned in the dental chair with the patient's mouth open and the lower jaw substantially immobilized by the jaw gripper device 33, probe 59 (Fig. 5) is attached to the hand gripper device 57 and is used to precisely locate the two reference points 76, 77 in the two jaws of the patient, so as to provide reference points for controlling the movements of the tool holder 58 during the actual dental treatment. Even though the jaw of the patient being treated is substantially immobilized, nevertheless it will usually be desirable to provide a 3-dimensional space locating system sensing any movements of the reference points 76, 77, during the actual dental treatment, to thereby enable precise control of the dental tool during the dental treatment. Such a space locating system is schematically indicated ---- box 82 in Fig. 4.

It will thus be seen that the dentist, while occupying workstation 60, can control all the operations involved in the dental treatment. These operations include controlling the chair motors CM₁₋ CM₆ (box 83, Fig. 4), and controlling the robotic arm motors AM₁ - AM₅ (box 84). Most of those operations would be controlled by the pre-prepared patient data as indicated by box 72 in Fig. 4, and the pre-prepared dental operational data as indicated by box 80 in Fig. 4. However, each of the dental operations may also be controlled by the dentist continuously, or as needed, via the manual inputs 81. The arrangement is such that the routine or menial tasks can be performed substantially automatically according to the inputted pre-prepared patient data and dental operation data, while the dentist still exercises overall control via the manual inputs 81, particularly via the joystick 63, of each operation involved in the particular dental treatment.

All data inputted into the system, as well as all operations performed by the system, are viewable in a real-time manner via the monitor 64. All records of the treatment may be archived, as indicated by box 85, Fig. 4, for record or future use purposes.

During the dental treatment, the video cameras 33 records all the dental operations performed, and this information may also be archived. The output of video camera 33 may also be observed by the dentist via monitor 64, and/or by the patient via screen 32.

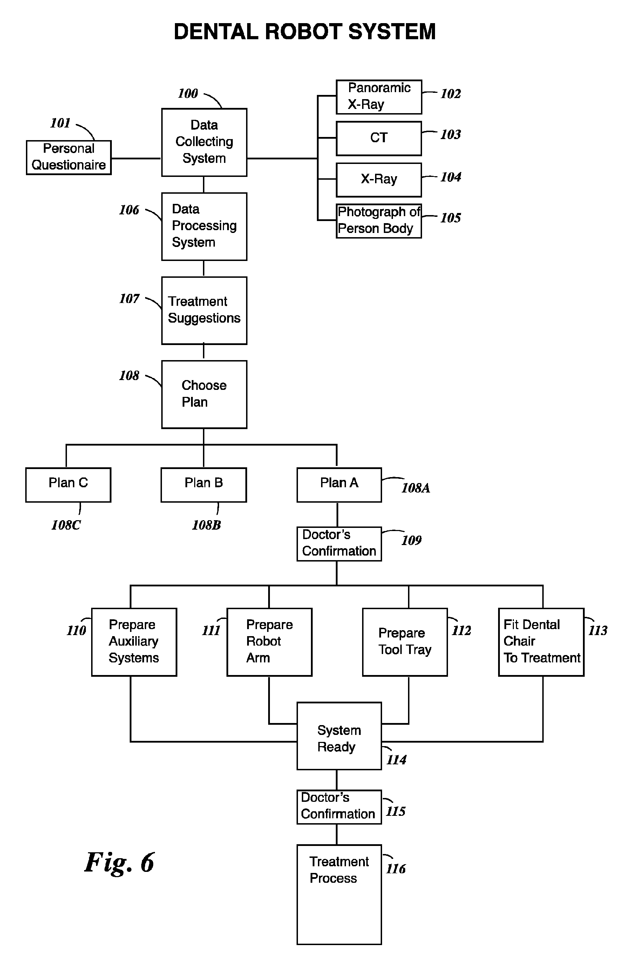

Fig. 6 is a block diagram illustrating a typical procedure that may be followed while using the computer controlled dental robotic system described above. The procedures illustrated in Fig. 6 is divided into two phases, namely: a data preparation phase, followed by a dental treatment phase. The data preparation phase involves storing in a first database patient data regarding the physical dimensions of the patient to be treated relevant to the selected dental treatment to be rendered, and storing in a second database control data regarding controls to be applied to the robotic arm 5 for rendering the selected dental treatment to the patient. The dental treatment phase involves utilizing the data from the first and second databases for automatically controlling the robotic arm 5 to manipulate the dental tool carried by the tool holder 58 in accordance with the patient data in the first database and the control data in the second database.

Thus, as shown in Fig. 6, the personal patient data is collected in a data collecting system 100 via a personal questionnaire 101, and also via a panoramic X-ray 102. a CT imaging system 103, an X-ray imaging system 104, photographs of the person's body 105, or any other suitable means for collecting the appropriate data. The collected data is suitably processed (block 106), together with various treatment suggestions (block 107), and a particular plan is chosen (block 108) among, e.g. three possible plans 108A, 108B, 108C. The selected plan is subject to the confirmation of the dentist (block 109).

Once a plan has been selected, all the necessary preparations are made, including preparing the auxiliary systems (block 110), preparing the robotic arm 5 (block 111), preparing the tool tray 4 (block 112) and preparing the dental chair 2 according to the selected treatment (block 113). When all the preparations have been made (block 114), and again confirmed by the dentist (block 115), the treatment process is initiated and is controlled by the data process system 72, Fig. 3, (block 116).

As indicated earlier, the dentist can, at any time, take-over control of any of the dental operations from the workstation 60 or, if necessary, can effect manual control by grasping the hand grip 57. The described system --- enables the routine or menial tasks to be performed substantially automatically, while the dentist still exercises overall control.

It will be appreciated that the system could be implemented in a modular manner, such that an operable system could be built of a relatively simple inexpensive construction, while permitting the system to be expanded, as needed, to add additional features and functions.

Many other variations, modifications and applications of the invention will be apparent.

## Claims

1. A dental treatment system, comprising:
a dental chair for receiving a patient to be treated, and including a head rest for receiving the head of the patient;
a robotic arm carrying a dental tool holder for holding a dental tool to be used in rendering a selected dental treatment, and for manipulating the dental tool with respect to the patient's mouth during the dental treatment; and
a control system programmable to control said robotic arm to manipulate the dental tool in accordance with the selected dental treatment to be rendered to the patient.

2. The dental treatment system according to Claim 1, wherein said dental chair further includes a jaw-engageable device engageable with the lower jaw of a patient on said dental chair to retain said lower jaw in a desired open position during a dental treatment.

3. The dental treatment system according to Claim 1, wherein said robotic arm further includes a hand grip graspable by a dentist for manually manipulating the dental tool holder, and the dental tool held thereby, independently of said control system.

4. The dental treatment system according to Claim 1, wherein said dental tool holder is carried by said robotic arm so as to be movable about at least five axes of movement.

5. The dental treatment system according to Claim 1, wherein said robotic arm includes:
a base;
a vertical post mounted on said base;
a first horizontal arm rotatably mounted to said vertical post about a vertical axis;
a second horizontal arm slidably mounted to said first horizontal arm;
a first pivotal arm pivotally mounted to one end of said second horizontal arm about a horizontal axis;
a second pivotal arm pivotally mounted at one end to the opposite end of said first pivotal arm about a pivotal axis;
a hand grip pivotally mounted about a pivotal axis at the opposite end of said second pivotal arm; and
a tool holder clamped to said third hand grip.

6. The dental treatment system according to Claim 1, wherein the system further includes a probe attachable to said robotic arm for locating preselected reference points in patients' mouths to serve as reference points in the control of said robotic arm by said control system.

7. The dental treatment system according to Claim 1, wherein said dental chair further includes a pair of ear-engageable members positionable to engage the ears of a patient on said dental chair; said ear-engageable members including ear pads for muting noise from the patient's ears, and/or for transmitting music to the patient's ears, during a dental treatment.

8. The dental treatment system according to Claim 1, wherein said dental chair further includes a television screen configured for application to the forehead to cover the eyes of a patient on said dental chair.

9. The dental treatment system according to Claim 1, wherein said system further includes a dental tray for holding various dental tools for convenient access by the dental tool holder carried by said robotic arm.

10. The dental treatment system according to Claim 9, wherein said control system is also programmable to control said robotic arm to automatically engage its dental tool holder with the appropriate dental tool carried by said tray in accordance with the selected dental treatment to be rendered to the patient.

11. The dental treatment system according to Claim 1, wherein said control system includes:
a first database storing patient data regarding the physical dimensions of the patient relevant to a dental treatment to be rendered to the patient;
a second database storing robotic arm control data regarding the movements to be applied to said robotic arm for rendering a dental treatment to the patient; and
a processor utilizing data from said first and second databases for controlling said robotic arm to manipulate the dental tool in accordance with the patient data in said first database and the control data in said second database for rendering the selected dental treatment to the respective patient on the dental chair.

12. A method of rendering a selected dental treatment to a patient, comprising:
providing a dental chair for the patient to receive the dental treatment, and a robotic arm carrying a dental tool to be used in rendering a selected dental treatment to the patient;
in a data preparation phase, storing in a first database patient data regarding the physical dimensions of the patient to be treated relevant to the selected dental treatment to be rendered, and storing in a second database control data regarding controls to be applied to the robotic arm for rendering the selected dental treatment to the patient; and
in a dental treatment phase, utilizing the data from said first and second databases for automatically controlling the robotic arm to manipulate the dental tool in accordance with the patient data in the first database and the control data in the second database for rendering the selected dental treatment to the respective patient on the dental chair.

13. The method according to Claim 12, wherein the patient data stored in said first database in the data preparation phase includes a reference mark in the patient's jaw to receive the dental treatment; and wherein said reference mark is sensed in the dental treatment phase and is utilized in controlling said robotic arm in the dental treatment phase.

14. The method according to Claim 13, wherein said reference mark is a metal dental element existing in the patient's jaw.

15. The method according to Claim 13, wherein said reference mark is a machine-sensible element applied for reference purposes to the patient's jaw.

16. The method according to Claim 13, wherein, in the data preparation phase, a reference mark in each of the patient's jaws is stored in said first database.

17. The method according to Claim 13, wherein said reference mark is sensed in the dental treatment phase by a manually manipulatable probe.

18. The method according to Claim 12, wherein, in the data preparation phase, relevant physical dimensions of the patient are stored in said first database by means of an electronic camera, a CT imaging system, an X-ray imaging system, and/or a panoramic imaging system.
